(19) Europäisches Patentamt
European Patent Office
Office européen des brevets

(11) Publication number: **0 009 866**
**B1**

(12) **EUROPEAN PATENT SPECIFICATION**

(45) Date of publication of patent specification: **04.08.82**

(21) Application number: **79301563.7**

(22) Date of filing: **03.08.79**

(51) Int. Cl.³: **C 07 C 103/38,**
C 07 C 103/78,
C 07 C 149/23,
C 08 K 5/20, C 08 K 5/37
//C07C93/187, C07D263/14,
C08L23/00

(54) **Phenolic esteramide antioxidants and their use.**

(30) Priority: **04.08.78 US 931087**
**30.05.79 US 43761**
**30.05.79 US 43771**
**30.05.79 US 43773**
**30.05.79 US 43789**
**30.05.79 US 43914**
**30.05.79 US 43954**

(43) Date of publication of application:
**16.04.80 Bulletin 80/8**

(45) Publication of the grant of the patent:
**04.08.82 Bulletin 82/31**

(84) Designated Contracting States:
**AT BE CH DE FR GB IT NL**

(56) References cited:
**BE - A - 546 134**
**DE - A - 2 014 165**
**DE - A - 2 018 716**
**DE - A - 2 021 133**
**DE - A - 2 027 613**
**DE - A - 2 309 376**
**DE - A - 2 445 345**
**DE - A - 2 512 895**
**US - A - 3 906 041**
**US - A - 3 927 091**
**US - A - 4 145 556**

(73) Proprietor: **UNIROYAL, INC.**
**1230 Avenue of the Americas Rockefeller Center**
**New York, New York 10020 (US)**

(72) Inventor: **Wheeler, Edward Lockwood**
**126 Claxton Avenue**
**Watertown Litchfield Connecticut (US)**
Inventor: **Jancis, Elmar Harry**
**89 Spruce Drive**
**Naugatuck New Haven Connecticut (US)**
Inventor: **Gencarelli, Richard Anthony**
**60 Maple Shade Road**
**Middletown Middlesex Connecticut (US)**
Inventor: **Barrows, Franklin Herbert**
**20 Stoddard Place**
**Beacon Falls New Haven Connecticut (US)**

(74) Representative: **Harrison, Michael Robert et al,**
**URQUHART-DYKES & LORD 11th Floor Tower House Merrion Way**
**Leeds LS2 8PB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 009 866 B1

# Phenolic esteramide antioxidants and their use

The present invention relates to novel phenolic esteramides which are useful in protecting organic materials such as synthetic and natural rubbers, petroleum products, and plastics from oxidative degradation.

As disclosed in German Patent Specification No. 2,445,345, bis(3,4-di-*t*-butyl-4-hydroxyphenyloxamido) alkanes have been proposed as polyolefin antioxidants.

The present invention relates to compounds of the formula $R^1COOANHM$, wherein A is $C_2$ to $C_{12}$ alkylene, $C_4$ to $C_8$ cycloalkylene or $—CH_2—(CH_2)_p—$, wherein p is an integer from 3 to 6, or A is $C_2$ to $C_5$ alkylene substituted with one or two $—CH_2OCO(CH_2)_mR^3$ groups, wherein $R^3$ is 3-W-5-Y-4-hydroxyphenyl, wherein W and Y may be the same or different and are $C_1$ to $C_{12}$ alkyl and m is 0,1 or 2; $R^1$ is $—(CH_2)_mR^3$, wherein m and $R^3$ are as defined above; and M is either hydrogen or $—COR^2$, wherein $R^2$ is $C_1$ to $C_{20}$ alkyl, $C_6$ to $C_{10}$ aryl or phenyl substituted with halogen, $C_1$ to $C_{12}$ alkyl, $C_1$ to $C_{12}$ alkoxy, nitro or hydroxy or $R^2$ is $—BCONHAOCOR^1$ wherein A and $R^1$ are as defined above and B is a single bond, $C_1$ to $C_{10}$ alkylene, phenylene, $C_4$ to $C_{12}$ oxydialkylene or $C_4$ to $C_{12}$ thiodialkylene, or $R^2$ is the same as $R^1$ which is defined above. The terms alkyl and alkylene comprise linear and branched moieties, the term cycloalkyl includes bridged groups having up to 8 carbon atoms.

A process for preparing a compound having the formula $R^1COOANHCOR^2$, wherein A is $C_2$ to $C_{12}$ alkylene, $C_4$ to $C_8$ cycloalkylene, or $—CH_2(CH_2)_p—$ wherein p is an integer from 3 to 6, or A is $C_2$ to $C_5$ alkylene substituted with one or two $—CH_2OCO(CH_2)_mR^3$ groups, wherein m is 0, 1 or 2 and $R^3$ is 3-W-5-Y-4-hydroxyphenyl wherein W and Y may be the same or different and are $C_1$ to $C_{12}$ alkyl; $R^1$ is $—(CH_2)_mR^3$, wherein m and $R^3$ are as defined above, and $R^2$ is $C_1$ to $C_{20}$ alkyl, $C_5$ to $C_6$ cycloalkyl, $C_6$ to $C_{10}$ aryl, phenyl substituted with halogen, $C_1$ to $C_{12}$ alkyl, $C_1$ to $C_{12}$ alkoxy, nitro or hydroxy, or $R^1$ and $R^2$ are $—(CH_2)_mR^3$ wherein m and $R^3$ are defined above, and B is a single bond, $C_1$ to $C_{10}$ alkylene, $C_4$ to $C_8$ cycloalkylene, phenylene, $C_4$ to $C_{12}$ oxydialkylene or $C_4$ to $C_{12}$ thiodialkylene; comprises reacting a compound of the formula $R^1COOANH_2$ with a compound of the formula $R^4COX$ or $B(COX)_2$, wherein A, $R^1$ and B are as defined above, X is halogen, hydroxy or $C_1$ to $C_4$ alkoxy and $R^4$ is $—(CH_2)_mR^3$, wherein m and $R^3$ are as defined above, $C_1$ to $C_{20}$ alkyl, $C_4$ to $C_8$ cycloalkyl, $C_6$ to $C_{10}$ aryl or phenyl substituted with halogen, $C_1$ to $C_{12}$ alkyl, $C_1$ to $C_{12}$ alkoxy, nitro, or hydroxy, said process being carried out at a temperature from 15°C to 250°C and at a pressure of 100Pa to 10kPa, with the proviso that if X is halogen, then an acid acceptor is employed in the reaction.

The present invention also relates to a process for preparing a compound having the formula $R^1COOANHCOR^2$, wherein A is $C_2$ to $C_5$ alkylene, $C_2$ to $C_5$ alkylene substituted with one or two $—CH_2OCO(CH_2)_mR^3$ groups, wherein $R^3$ is 3-W-5-Y-4-hydroxyphenyl, wherein W and Y may be the same or different and are each $C_1$ to $C_{12}$ alkyl, and m is 0, 1 or 2, or A is $C_4$ to $C_8$ cycloalkylene or $—CH_2(CH_2)_p—$ wherein p is an integer from 3 to 6; and $R^1$ is $—(CH_2)_mR^3$, and $R^2$ is $C_1$ to $C_{20}$ alkyl, $C_6$ to $C_{10}$ aryl, or phenyl substituted with halogen, $C_1$ to $C_{12}$ alkyl, $C_1$ to $C_{12}$ alkoxy, nitro or hydroxy, or $R^2$ is $BCONHAOCOR^1$ wherein A and $R^1$ are as defined above and B is a single bond, $C_1$ to $C_{10}$ alkylene, phenylene, $C_4$ to $C_{12}$ oxydialkylene or $C_4$ to $C_{12}$ thiodialkylene; comprising reacting a compound of the formula

$$R^4\overset{O}{\overset{\|}{C}}—NH—Q—(OH)_s$$

or

$$B[—\overset{O}{\overset{\|}{C}}NH—Q—(OH)_s]_2$$

with a compound of the formula $R^1COX$ wherein Q is $C_2$ to $C_{12}$ alkylene, $C_4$ to $C_8$ cycloalkylene or $—CH_2—(CH_2)_p—$, s is an integer from 1 to 3, and B, $R^1$, X and p are as defined above and $R^4$ is $—(CH_2)_mR^3$, wherein m and $R^3$ are as defined above, $C_1$ to $C_{20}$ alkyl, $C_5$ to $C_6$ aryl or phenyl substituted with halogen, $C_1$ to $C_{12}$ alkyl, $C_1$ to $C_{12}$ alkoxy, nitro or hydroxy, said process being carried out at a temperature from 15°C to 250°C and at a pressure of 100Pa to 10kPa, with the proviso that if X is halogen then an acid acceptor is employed in the reaction.

The present invention also relates to compositions comprising the compounds of the present invention and organic materials subject to oxidative degradation.

A is preferably alkylene having terminal $CH_2$— groups. Most preferably the ester and amide groups are attached to alpha $CH_2$-terminated alkylene not having hydrogen on the carbon atoms in the beta-position.

The preferred definition of $R^2$ depends to some extent on the projected use of the product. When high hydrocarbon solubility is desired $R^2$ should be a long chain hydrocarbon having 11 to 17 carbon

atoms. In a particularly useful compound $R^2$ is $C_{17}H_{35}$. If low volatility is desired, $R^2$ is preferably $BCONHAOCOR^1$, wherein B, A and $R^1$ are as defined above. Particularly useful are compounds wherein B is a single covalent bond. Particularly high molecular weights and low volatilities can be achieved when $R^2$ is $BCONHAOCOR^1$ wherein A is an alkylene substituted with $—CH_2OCO(CH_2)_mR^3$ wherein m and $R^3$ are as defined above. The preferred halogens are fluorine, chlorine, bromine and iodine.

The compounds of the present invention are useful in stabilizing organic materials normally subject to oxidative degradation. Materials that are thus stabilized include a multitude of synthetic polymers. Among those polymers are various polyolefins such as polyethylene, polypropylene, polybutylene, polybutadiene, polymethylpentene. Other polymers stabilized by the compounds of the present invention include acetal resins, polyacrylates, polymethacrylates, polydialkylphthalate, cellulosics, polyamides, polyesters, polyurethanes, polycarbonate, polystyrene, polyvinyl chloride, polyvinylidene chloride. Copolymers can also be stabilized by the compounds of the present invention. Representative copolymers include ethylene/propylene copolymers, butadiene/styrene copolymers, ethylene/vinyl acetate copolymers, and ethylene/ethyl acrylate copolymers. Copolymers also include terpolymers such as ethylene/propylene/non-conjugated diene terpolymers and acrylonitrile/butadiene/styrene interpolymers. Polymer blends such as polystyrene/polyphenylene oxide and ethylenepropylene copolymer or terpolymer/polypropylene can also be stabilized by the compounds of the present invention. Other materials stabilized by compounds of the present invention include hot melt adhesives such as those based on polyesters, polyamides or ethylene/vinyl acetate. Also stabilized are petroleum products such as fuels, lubricating oils, petrolatum jellies, and natural products such as natural rubber, waxes, fat, tallow, linseed oil, corn oil, cottonseed oil, and codliver oil. The preceding list is representative, though by no means exhaustive, of the products that can benefit from the compounds of the present invention. To achieve protection against oxidative degradation, the compounds of the present invention are added in the amounts generally used for known antioxidants which may have similar properties to achieve such protection. Depending on the substrate used, the antioxidant is added in amounts of 0.001 to 10 percent by weight based on the weight of the substrate, with the usual range being from 0.05 to 2.0 percent.

The compounds of the present invention can be used by themselves to stabilize organic materials, or they can be used in combination with other stabilizers. Such other stabilizers might include other phenolics, thio compounds of various kinds, such as thiodipropionate esters, phosphites and phosphonates, anti-copper chemicals such as oxalamides, ultraviolet stabilizers of various kinds as well as other additives where the use of such additives has been found beneficial.

The compounds of the present invention can generally be made from known starting materials by amidification and esterification reactions well known in the literature. Convenient aminoalcohols used in the preparation of the compounds of the present invention include: ethanolamine, 2-aminopropanol, 2-amino-2-methyl-1-propanol, 3-amino-2-methyl-1-propanol, 2-amino-2-methyl-1,3-propanediol, 2-amino-2-ethyl-1,3-propanediol, 2-amino-1-butanol, tris(hydroxymethyl)aminomethane, 1-amino-1-cyclopentanemethanol, 1-aminomethyl-1-cyclohexanemethanol, 6-amino-1-hexanol, 2-amino-3-methyl-1-butanol, 5-amino-1-pentanol, 3-amino-1,2-propanediol, 3-amino-1-propanol, 2-aminocyclohexanol, 4-amino-cyclohexanol, 3-amino-2-butanol, 1-amino-2-dodecanol, 2,2-di-methyl-3-amino-1-propanol, 2-aminomethyl-2-methyl-1,3-propanediol, 2,2,2-tris-(hydroxymethyl)aminoethane. Many other aminoalcohols are readily made by known methods from available starting materials.

A convenient synthesis of a compound of the present invention would generally include making the amide from one of the aforementioned alkanolamines followed by esterification of the hydroxyalkylamide. The amide can readily be prepared by reacting the alkanolamine with an appropriate acid, acid chloride or an ester. Suitable acids include $C_1$ to $C_{21}$ aliphatic acids, benzoic acid, substituted benzoic acids such as alkylbenzoic acid, chlorobenzoic acid, dialkylbenzoic acid, 4-hydroxy-3,5-di-alkylbenzoic acid, 2-(4-hydroxy-3,5-di-alkylphenyl)acetic acid or 3-(4-hydroxy-3,5-di-alkylphenyl)propionic acid.

When the bisamides are desired, then suitable acids include oxalic acid, phthalic acid, terephthalic acid, thiodialkanoic acid and aliphatic diacids of the structure $HO—CO—CnH_{2n}—COOH$ where n is an integer from 1 to 10. Examples of such acids are malonic, succinic, adipic, palmitic, azaleic, sebacic and others. The esters and acid chlorides of all of the abovementioned acids are likewise suitable starting materials in making amides. It is recognized that imides can form from some of the aforementioned acids. Suitable precautions, such as use of milder conditions and a large excess of amine to maximize amide formation, are advisable in such instances.

The usual starting material for esterification is a 3,5-dialkyl-4-hydroxybenzoic acid, a 2-(3,5-dialkyl-4-hydroxyphenyl)acetic acid or a 3-(3,5-dialkyl-4-hydroxyphenyl)propionic acid, its acid chloride or ester.

If an esteramide is desired where $R^1$ and $R^2$ are identical, then the esterification and amidification can be carried out in one step.

When the starting amine is ethanolamine, substituted with alkyl or hydroxyalkyl, then a convenient alternative to the above methods is available. This involves forming an oxazoline from the acid or its derivative and the amino alcohol according to known methods. The oxazoline can then be hydrolized to an aminoester, whcih in turn can be amidified according to known procedures.

The preparations indicated above are mentioned as non-limiting examples of ways to carry out the objects of this invention. Other methods will suggest themselves to those skilled in the art.

Of the following Examples, those numbered 1, 4, 6, 9, 11, 21, 27, 31, 35 and 37 illustrate the preparation of starting materials. Other Examples illustrate the preparation and use of the compounds of the present invention.

Example 1

*3-(3,5-di-tert-butyl-4-hydroxyphenyl)-N-(2-hydroxyethyl)propionamide*

In a 3 liter, three-neck, round-bottom flask equipped with a thermometer, stirrer and Stark and Dean trap with a condenser was placed 556 g (2.0 moles) of 3-(3,5-di-t-butyl-4-hydroxyphenyl)propionic acid, 135 g (2.2 moles) of ethanolamine and 500 ml of xylene. The mixture was refluxed for 11 hours and 79 ml of azeotrope was collected. The xylene was removed at reduced pressure and the residue dissolved in hot toluene. The title compound (211 g) which crystallized on cooling melted at 129—130°C.

Example 2

*2-[3-(3,5-di-tert-butyl-4-hydroxyphenyl)propionamido]ethyl laurate*

A mixture of 44 g (.22 mole) lauric acid, 64 g (0.2 mole) of 3(3,5-di-*tert*-butyl-4-hydroxyphenyl)-N-(2-hydroxyethyl)propionamide, 2 g Tyzor TBT (trademark, tetrabutyl titanate) catalyst and 100 ml xylene was refluxed for 5 hours. The water (3.8 ml) was collected in a Start & Dean trap. The xylene was removed by vacuum stripping. The residue was taken up in acetonitrile. After the solution was cooled in the freezer, a white precipitate formed. The actonitrile was removed by decantation and the recrystallization was repeated. The final traces of acetonitrile were removed by heating the product under vacuum and the title product (66 g) thus obtained was an amber oil at room temperature.

Example 3

*2-[3-(3,5-di-tert-butyl-4-hydroxyphenyl)propionamido]ethyl stearate*

A mixture of 32.1 g (0.1 mole) 3-(3,5-di-tert-butyl-4-hydroxyphenyl)-N-(2-hydroxyethyl)propionamide, 28.5 g (0.1 mole) stearic acid, 1 g Tyzor TBT catalyst, and 50 ml xylene was refluxed at 140—170°C until evolution of water ceased. The Stark & Dean trap captured 3.5 ml of water. The xylene was removed by vacuum stripping and the residue was crystallized from acetonitrile. The title compound (53 g) melted at 55—57°C.

Example 4

*N-(2-hydroxyethyl)lauramide*

To a solution of 24 g (0.4 mole) ethanolamine in 100 ml toluene was added 40 g (0.2 lauric acid at 50°C. The mixture was refluxed for 6 hours. Toluene was gradually removed to increase the final pot temperature to 140°C. The distillate was collected in a Stark & Dean trap. The toluene was removed by vacuum stripping and the residue was crystallized from n-hexane. The title compound (44 g) thus obtained melted at 80—84°C.

Example 5

*2-(lauramido)ethyl 3-(3,5-di-tert-butyl-4-hydroxyphenyl)propionate*

A mixture of 13.9 g (.055 mole) N-(2-hydroxyethyl)lauramide, 13.9 g (0.05 mole) 3(3,5-di-*tert*-butyl-4-hydroxyphenyl)propionic acid, 0.5 g Tyzor TBT catalyst and 100 ml xylene was refluxed until evolution of water ceased (3 hours). The xylene was removed by vacuum stripping. The residue was taken up in acetonitrile. White crystals separated upon cooling at about −10°C. The acetonitrile was decanted and the recrystallization was repeated. The last traces of acetonitrile were removed under vacuum, and the title product thus obtained was a yellow oil at room temperature.

Example 6

*N-(2-hydroxyethyl)stearamide*

To a solution of 24.4 g (0.4 mole) ethanolamine in 500 ml ethanol at 60°C was added 85.2 g (0.3 mole) stearic acid. The ethanol was distilled off until the pot temperature reached 100°C, at which time 300 ml toluene was added, and the water was distilled off as an azeotrope. After 5 hours at 110°C, 6 ml water was recovered in the Stark & Dean trap. Some toluene was removed to raise the pot temperature to 135°C. After an additional 4 hours, another 7 ml water was trapped. The title product (38.5 g) that crystallized on cooling had a melting point of 96—99°C.

Example 7

*2-stearamidoethyl 3-(3,5-di-tert-butyl-4-hydroxyphenyl)propionate*

A mixture of 30.6 g (0.11 mole) 3-(3,5-di-*tert*-butyl-4-hydroxyphenyl)propionic acid, 32.6 g (0.1 mole) N-(2-hydroxyethyl)stearamide, 0.8 g Tyzor TBT, and 100 ml xylene was refluxed for six hours. The water (1.8 ml) was trapped in a Stark & Dean trap. The solution was filtered through Microcel (trademark, synthetic calcium silicate) and the solvent was removed by vacuum stripping. The title compound (51 g) was recrystallized from acrylonitrile. It melted at 52—55°C.

Example 8

*2-[3-(3,5-di-tert-butyl-4-hydroxyphenyl)propionamido]ethyl 3-[3-(3,5-di-tert-butyl-4-hydroxyphenyl)-propionate*

A mixture of 32 g (0.1 mole) of 3-(3,5-di-*tert*-butyl-4-hydroxyphenyl)-N-(2-hydroxyethyl)-propionamide, 27 g (0.1 mole) of 3(3,5-di-*tert*-butyl-4-hydroxyphenyl)propionic acid, 1 g Tyzor TBT catalyst and 100 ml xylene was refluxed at 150° until evolution of water ceased. In the course of four hours, two ml water was collected in the Stark & Dean trap. The xylene was removed by vacuum stripping. The title compound (54 g) was recrystallized from a toluene/hexane mixture. It melted at 151—154°C.

Example 9

*2[2-(3,5-di-tert-butyl-4-hydroxyphenyl)ethyl]-4,4-dimethyl-2-oxazoline*

A mixture of 167 g (0.6 mole) 3-(3,5-di-*tert*-butyl-4-hydroxyphenyl)propionic acid, 65 g (1.06 mole) 2-amino-2-methylpropanol and 100 ml xylene was refluxed for six hours. The water was trapped in a Stark & Dean trap. The mixture was poured into 300 ml hexane, and upon cooling the crystalline product was filtered off. The title product (101 g) thus obtained melted at 138—141°C.

Example 10

*2-amino-2-methylpropyl [3-(3,5-di-tert-butyl-4-hydroxyphenyl)propionate]*

In a 1-liter, three-neck, round-bottom flask equipped with a thermometer, stirrer and condenser were placed 66.2 g (0.2 mole) 2-[2-(3,5-di-*tert*-butyl-4-hydroxyphenyl)ethyl]-4,4-dimethyl-2-oxazoline, 400 ml isopropanol and 33 ml 6N hydrochloric acid. The mixture was heated to 45°C for 2 hours and then cooled. To this stirred solution were added 250 ml of water and 33 ml of 6N sodium hydroxide. The product was removed by filtration and dried. The title compound, (52 g), thus obtained, melted at 97—99°C.

Example 11

*3-(3,5-di-tert-butyl-4-hydroxyphenyl)-N-(1,1-dimethyl-2-hydroxyethyl)propionamide*

In a 1-liter, three-neck, round bottom flask equipped with a thermometer, stirrer and condenser were placed 34.9 g (0.1 mole) 2-amino-2-methylpropyl [3-(3,5-di-*tert*-butyl-4-hydroxyphenyl)-propionate] and 200 ml isopropanol. The mixture was refluxed for 15 hours. The solvent was stripped off, and the residue was dissolved in hot hexane. The title compound (20 g) was removed by filtration and dried. It melted at 120—122°C.

Example 12

*2-[3-(3,5-di-tert-butyl-4-hydroxyphenyl)propionamido]-2-methylpropyl laurate*

A solution of 55 g (0.25) lauroyl chloride in 50 ml toluene was added dropwise to a solution of 70 g (0.2 mole) 3-(3,5-di-*tert*-butyl-4-hydroxyphenyl)-N-(1,1-dimethyl-2-hydroxyethyl)propionamide in 60 ml dimethylaniline and 50 ml toluene. The mixture was heated $4\frac{1}{2}$ hours at 50°C. The mixture was washed with dilute hydrochloric acid followed by a brine wash. The toluene was removed by vacuum stripping. The residue was taken up in acetonitrile. The solution was shaken with decolorizing charcoal, filtered, and then placed in a freezer. The title compound (20 g) crystallized and was removed by filtration. It melted at 24—27°C.

Example 13

*2-[3-(3,5-di-tert-butyl-4-hydroxyphenyl)propionamido]-2-methylpropyl palmitate*

In a 1-liter, three-neck, round-bottom flask equipped with a thermometer, stirrer and dropping funnel were placed 13 g (0.037 mole) of 3-(3,5-di-*tert*-butyl-4-hydroxyphenyl)-N-(1,1-dimethyl-2-hydroxyethyl)propionamide, 10 g triethylamine and 250 ml of benzene. To this stirred mixture was added 10.2 g (0.037 mole) of palmitoyl chloride, and the mixture was stirred for 3 hours. The mixture was filtered and stripped and the residue dissolved in hot acetonitrile. Some palmitic anhydride crystallized out, and it was filtered off. The acetonitrile solution was refrigerated overnight and the title compound (20 g) crystallized. It was removed by filtration and then dried. It melted at 41—43°C.

Example 14

*2-palmitamido-2-methylpropyl 3-(3,5-di-tert-butyl-4-hydroxyphenyl)-propionate*

In a 1-liter, three-neck, round-bottom flask equipped with a thermometer, stirrer and dropping funnel were placed 17.5 g (0.05 mole) of 2-amino-2-methylpropyl 3-(3,5-di-t-butyl-4-hydroxyphenyl)-propionate, 500 ml of benzene and 10 g of triethylamine. To this stirred mixture was added 14 g (0.05) of palmitoyl chloride and the mixture stirred for four hours. The mixture was filtered and stripped, and the residue dissolved in hot acetonitrile. After being stored for about 4 days at about 5°C, the product crystallized. It was removed by filtration and dried. The title compound, (14 g) thus obtained, melted at 43—46°C.

Example 15

*2-[3-(3,5-di-tert-butyl-4-hydroxyphenyl)propionamido]-2-methylpropyl stearate*

In a 1-liter, three-neck, round-bottom flask equipped with a thermometer, stirrer and dropping funnel were placed 26.2 g (0.075 mole) of 3-(3,5-di-*tert*-butyl-4-hydroxyphenyl)-N-(1,1-dimethyl-2-hydroxyethyl)propionamide, 20 g triethylamine and 400 ml benzene. To this stirred mixture was added 22.5 g (0.075 mole) of stearoyl chloride, and the reaction was stirred for 18 hours. The triethylamine hydrochloride was removed by filtration, and the solvent was stripped off. The residue was dissolved in hot hexane, and 2.5 g of stearic anhydride was isolated. The hexane was stripped off, and the residue was dissolved in hot acetonitrile. The title compound crystallized on cooling. It was removed by filtration and was dried. It melted at 59—61 °C.

Example 16

*2-stearamido-2-methylpropyl 3-(3,5-di-tert-butyl-4-hydroxyphenyl)propionate*

In a 500 ml, three-neck, round-bottom flask equipped with a thermometer, stirrer and dropping funnel were placed 26 g (0.075 mole) of 2-amino-2-methylpropyl 3-(3,5-di-t-butyl-4-hydroxyphenyl)propionate, 200 ml of benzene and 8.2 g of triethylamine. To this stirred mixture was added 22.5 g (0.075 mole) of stearoyl chloride, and the mixture stirred for 4 hours. The triethylamine hydrochloride was removed by filtration and the solvent was stripped off. The residue was dissolved in hot hexane. After crystallization, filtration and drying, the title compound melted at 46—48°C.

Example 17

*2 - (3,5 - di - tert - butyl - 4 - hydroxybenzamido) - 2 - methylpropyl 3 - (3,5 - di - tert - butyl - 4 - hydroxyphenyl)propionate*

In a 1-liter, three-neck, round-bottom flask equipped with a thermometer, stirrer and dropping funnel were placed 34.9 g (0.1 mole) of 2-amino-2-methylpropyl 3-(3,5-di-*tert*-butyl-4-hydroxyphenyl)propionate, 20 g of triethylamine and 400 ml of benzene. To this stirred mixture was added 26.8 g (0.094 mole) of 3,5-di-t-butyl-4-hydroxybenzoyl chloride, and the mixture was stirred overnight. The mixture was filtered and stripped, and the residue was slurried with hexane. The product was filtered off and recrystallized from isopropanol. The title compound (30.5 g) thus obtained melted at 192—194°C.

Example 18

*2 - [3 - (3,5 - di - tert - butyl - 4 - hydroxyphenyl)propionamido]iso - butanetriyl tris[3 - (3,5 - di - tert - butyl - 4 - hydroxyphenyl)propionate]*

A solution of 85 g (0.27 mole) 3(3,5-di-*tert*-butyl-4-hydroxyphenyl)propionyl chloride in 120 ml toluene was added to 7.3 g (0.06 mole) tris(hydroxymethyl)aminomethane in 60 ml pyridine and 30 ml toluene. The mixture was stirred 42 hours at 60°C. The mixture was washed with dilute acid and then with brine. The toluene was removed by vacuum stripping and the residue was crystallized in n-hexane. The title compound thus obtained melted at 118—122°C.

| Elemental Analysis: | | Calculated | Found |
|---|---|---|---|
| | C | 74.37 | 74.19 |
| | H | 9.28 | 9.47 |
| | N | 1.21 | 1.11 |

Example 19

*2 - [2 - (3,5 - di - tert - butyl - 4 - hydroxyphenyl)ethyl] - 2 - oxazoline - 4,4 - dimethyl bis[3 - (3,5 - di - tert - butyl - 4 - hydroxyphenyl)propionate*

A mixture of 167 g (0.6 mole) 3-(3,5-di-*tert*-butyl-4-hydroxyphenyl)propionic acid, 24.2 g (0.2 mole) tris(hydroxymethyl)aminomethane and 100 ml xylene were refluxed for 6 hours. The water was collected in a Stark and Dean trap. The hot mixture was poured hot into 300 ml hexane. The title product precipitated. It was recrystallized from benzene to give the title compound (166.5 g) melting at 173—175°C.

| Elemental Analysis: | | Calculated | Found |
|---|---|---|---|
| | C | 74.70 | 74.53 |
| | H | 9.23 | 9.09 |
| | N | 1.58 | 1.34 |

Example 20

*2-stearamidoisobutanetriyl tris[3-(3,5-di-tert-butyl-4-hydroxyphenyl)propionate*

To 44.2 g (0.05 mole) of 2-[3-(3,5-di-tert-butyl-4-hydroxyphenyl)ethyl]-2-oxazoline-4,4-dimethyl bis[3-(3,5-di-*tert*-butyl-4-hydroxyphenyl)propionate] in 200 ml toluene was added 8.2 ml 6N hydrochloric acid. The mixture was stirred for 4 hours, and 8 g sodium bicarbonate was then added. The water layer was separated, and to the organic layer was added 40 ml triethylamine, followed by 15.1 g (0.05 mole) stearoyl chloride in 30 ml toluene. This mixture was stirred overnight at room temperature and was then stirred one hour at 50°C. Upon cooling, the mixture was washed first with dilute hydrochloric acid and then with brine. The toluene was removed by vacuum stripping. The residue was purified by passing it through a silica column using 50/50 (volume) toluene/hexane mixture as the eluent. The title compound (28 g) obtained after evaporation of the eluent, melted at 77—79°C.

Example 21

*N,N'-bis(hydroxyethyl)oxamide*

To a solution of 134 g (0.22 mole) ethanolamine in 1 liter of ethanol was added 146 g (0.1 mole) diethyl oxalate in the course of one hour. The mixture was heated for one hour at 50°C. The title compound (162 g) precipitated upon cooling. After filtration and drying, it melted at 166—168°C.

Example 22

*2,2'-oxalyldiamidobis[ethyl 3-(3,5-di-tert-butyl-4-hydroxyphenyl)propionate]*

A mixture of 55.6 g (0.2 mole) of 3-(3,5-di-tert-butyl-4-hydroxyphenyl)propionic acid, 17.6 g (0.1 mole) of N,N'-bis (2-hydroxyethyl)oxamide, 1.5 g Tyzor TBT, and 200 ml xylene was refluxed. The water and xylene were removed until the pot temperature reached 200°C. Upon cooling the title compound (54 g) crystallized out. After recrystallization from toluene, it melted at 128—131°C.

Example 23

*2,2'-oxalyldiamidobis[2-methylpropyl 3-(3,5-di-tert-butyl-4-hydroxyphenyl)propionate]*

In a 500 ml, three-neck round-bottom flask equipped with a thermometer, stirrer and dropping funnel were placed 35 g (0.1 mole) of 2-amino-2-methylpropyl 3-(3,5-di-t-butyl-4-hydroxyphenyl)-propionate, 250 ml of benzene and 20 g of triethylamine. To this stirred mixture was added 6.35 g (0.05 mole) of oxalyl chloride, and the mixture was stirred for 2 hours. The mixture was filtered, and the solvent was stripped off. The residue was dissolved in a 2:1 mixture of benzene/hexane and allowed to cool. After the crystalline product (22 g) was recrystallized from a 2:1 mixture of benzene/hexane, it melted at 169—171°C.

Example 24

*2,2'-adipamidobis-[2-methylpropyl 3-(3,5-di-tert-butyl-4-hydroxyphenyl)propionate]*

In a 1-liter, three-neck, round-bottom flask equipped with a thermometer, stirrer and dropping funnel were placed 29 g (0.083 mole) of 2-amino-2-methylpropyl [3-(3,5-di-t-butyl-4-hydroxyphenyl)propionate], 20 g of triethylamine and 400 ml benzene. To this stirred mixture was added 7.6 g (0.0415 mole) of adipoyl chloride, and the mixture was stirred for 4 hours. The mixture was filtered and stripped, and the residue dissolved in hot hexane. The product crystallized and was then recrystallized from a mixture of 400 ml of isooctane-125 ml of toluene. The purified product (21 g) melted at 135—138°C.

Example 25

*2,2'-sebacamidobis-[2-methylpropyl 3-(3,5-di-tert-butyl-4-hydroxyphenyl)propionate]*

In a 1-liter, three-neck, round-bottom flask equipped with a thermometer, stirrer and dropping funnel were placed 34.9 g (0.1 mole) of 2-amino-2-methylpropyl 3-(3,5-di-t-butyl-4-hydroxyphenyl)propionate, 20 g of triethylamine and 400 ml of benzene. To this stirred mixture was added 11.9 g (0.05 mole) of sebacoyl chloride, and the mixture was stirred for 5 hours. The mixture was filtered and stripped, and the residue was dissolved in hot hexane. A solid separated and it was recrystallized from hexane/benzene (3:1). The title compound (32 g) thus obtained melted at 113—117°C.

Example 26

*2,2'-sebacamidobis[isobutanetriyl tris[3-(3,5-di-tert-butyl-4-hydroxyphenyl)propionate]]*

To 44.2 g (0.05 mole of 2-[2-(3,5-di-tert-butyl-4-hydroxyphenyl)ethyl]-2-oxazoline-4,4-dimethyl bis[3-(3,5-di-*tert*-butyl-4-hydroxyphenyl)propionate] is 200 ml toluene was added 8.2 ml 6N hydroxhloric acid. The mixture was stirred for 3 hours, and 6 g sodium bicarbonate was then added. The mixture was stirred 15 minutes, and the layers were separated. To the organic layer was added 40 ml triethylamine followed by 6 g (0.025 mole) sebacoyl chloride in 30 ml toluene. This mixture was stirred overnight at room temperature, and was then stirred one hour at 50°C. Upon cooling, the mixture was washed first with dilute hydrochloric acid and then with brine. The toluene was removed by vacuum stripping. The residue was taken up in 1:1 toluene/hexane and passed through a fluorosil column. The title compound (11 g), isolated after removal of solvent, melted at 134—141°C.

Example 27

*N,N'-bis[1,1-bis(hydroxymethyl)-2-hydroxyethyl]terephthalamide*

In a 1-liter, three-neck, round-bottom flask equipped with a thermometer, stirrer and condenser were placed 194 g (1.0 mole) of dimethyl terephthalate, 242 g (2.0 mole) tris(hydroxymethyl)aminomethane and 1 liter of methanol. The mixture was refluxed for 8 hours and then allowed to cool. The title compound crystallized and was removed by filtration and dried. The melting point was 188—190°C.

Example 28

*2,2'-terephthalamidobis[isobutanetriyl tris[3-(3,5-di-tert-butyl-4-hydroxyphenyl)propionate]]*

In a 1-liter, three-neck, round-bottom flask equipped with a thermometer, stirrer, dropping funnel and condenser were placed 18.6 g (0.05 mole) of N,N'-bis[1,1-bis(hydroxymethyl)-2-hydroxyethyl]terephthalamide and 200 ml of pyridine. To this stirred slurry was added 90 g (0.3 mole) of 3-(3,5-di-t-butyl-4-hydroxyphenyl)propionyl chloride at 20—30°C, and the mixture was stirred for 18 hours. The mixture was filtered and stripped, and the residue was dissolved in hot cyclohexane. Trace amounts of pyridine hydrochloride were filtered off and the solution was allowed to cool. The title product (61 g) crystallized on cooling. After recrystallization from methanol, it melted at 169—172°C.

Example 29

*3,3'-Thiobis[2-propionamidoethyl-3-(3,5-di-tert-butyl-4-hydroxyphenyl)propionate*

A solution of 26.8 g (0.44 mole) ethanolamine and 41.2 g (0.2 mole) dimethyl 3,3'-thiodipropionate in 100 ml ethanol was refluxed for 5 hours. The 3,3'-thiobis-[N-(2-hydroxyethyl) propionamide] precipitated as a white crystalline solid and upon washing with acetone melted at 130—132°.

A mixture of 18 g (0.068 mole) at the aforementioned intermediate, 41.7 g (0.15 mole) 3-(3,5-di-*tert*-butyl-4-hydroxyphenyl)propionic acid and 1 g Tyzor TBT catalyst in 100 ml xylene was refluxed until evolution of water ceased. 3.5 ml of water was recovered in the Stark and Dean trap. Upon evaporation of the xylene, the product (51 g) was obtained as a viscous yellow oil.

Example 30

*3,3'-thiobis[2-methyl-2-propionamidopropyl-3-(3,5-di-tert-butyl-4-hydroxyphenyl)propionate]*

A mixture of 40 g (0.44 mole) 2-amino-2-methylpropanol, 3 g of potassium *tert*-butoxide and 41.2 g (0.2 mole) dimethyl 3,3'-thio-dipropionate was stirred for ½ hour at room temperature and was then heated to 90°C. for ½ hour under vacuum. The excess 2-amino-2-methylpropanol was removed by vacuum stripping. One-half (0.1 mole) of the resulting 3,3'-thiobis-N-(1,1-dimethyl-2-hydroxyethyl)propionamide was heated with 61.2 g (0.2 mole) ethyl 3-(3,5-di-*tert*-butyl-4-hydroxyphenyl)propionate and 2 g potassium *tert*-butoxide for 2 hours under vacuum at 100—110°C. The mixture was taken up in toluene, neutralized with acetic acid and washed with water. Upon removal of the toluene by vacuum distillation, the title compound (79 g) was obtained as a viscous yellow oil.

Example 31

*N,N'-bis[2-hydroxyethyl]terephthalamide*

A mixture of 194 g (1.0 mole) of dimethyl terephthalate, 150 ml (2.46 mole) ethanolamine and 300 ml ethanol was refluxed for 7 hours. On cooling the title product separated. It was filtered and then washed with ethanol. After drying, it melted at 227—229°C.

Example 32

*2,2'-terephthalamido-N,N'-bis[ethyl [3-(3,5-di-tert-butyl-4-hydroxyphenyl)propionate]]*

To a mixture of 25.2 g (0.1 mole) N,N'-bis(2-hydroxyethyl) terephthalamide and 200 ml pyridine was added 59.3 g (0.2 mole) of 3-(3,5-di-*tert*-butyl-4-hydroxyphenyl)propionyl chloride in 300 ml toluene. The mixture was stirred for 2 hours. It was then heated to 90°C and filtered hot. The solvent was removed by distillation and the residue was crystallized from n-hexane. The title product (68 g) thus obtained was filtered and then washed with hot water. After drying, it melted at 173—175°C.

Example 33

*3-oxalylamido-bis-[propyl 3-(3,5-di-tert-butyl-4-hydroxyphenyl)propionate]*

Diethyl oxalate (73 g; 0.5 mole) was added over the course of a half hour to a solution of 82.5 g (1.1 mole) 3-amino-1-propanol in 500 ml ethanol. The temperature rose to 50°C. The mixture was stirred for an hour and the resulting precipitate (m.p. 156—158°C) was separated by filtration. A portion (20.4 g) of the N,N'-(3-hydroxypropyl) oxamide thus obtained was added to a mixture of 61.2 g (0.22 mole) 3-(3,5-di-*tert*-butyl-4-hydroxyphenyl)propionic acid, 100 ml xylene and 2 g Tyzor TBT catalyst. The mixture was refluxed for four hours and then filtered through Microcel. The xylene was removed by vacuum distillation. The residue was taken up in hot hexane. The title compound crystallized on cooling and melted at 120—123°C.

| Elemental Analysis: | | Calculated | Found |
|---|---|---|---|
| | C | 69.61 | 69.96 |
| | H | 8.84 | 8.84 |
| | N | 3.87 | .4.02 |

Example 34

*3-stearamidopropyl 3(3,5-di-tert-butyl-4-hydroxyphenyl)propionate*

Stearic acid (56.8 g; 0.2 mole) was added to a hot (80°C) mixture of 30 g (0.4 mole) 3-aminopropanol, 100 ml xylene and 0.5 g sodium methoxide. The mixture was heated at reflux for two hours with removal of water and was then poured into hexane, in which N-(3-hydroxypropyl)stearamide crystallized and was obtained by filtration; it melted at 83—89°C. After the product was crystallized from acetone, melting point rose to 94—96°C. A 34.1 g (0.1 mole) portion of this intermediate was heated with 27.8 g (0.1 mole) of 3-(3,5-di-*tert*-butyl-4-hydroxyphenyl)propionic acid, 1 g of Tyzor TBT catalyst and 100 ml xylene. After the mixture was refluxed for 3 hours, 2 ml of water was collected in the Stark and Dean trap. The xylene was distilled off under vacuum, and the residue was crystallized from acetonitrile. The title product (29 g) thus obtained melted at 50—52°C.

Example 35

*N-(2,2-dimethyl-3-hydroxypropyl) stearamide*

In a 500 ml, three-neck, round-bottom flask equipped with a thermometer, stirrer and a Stark and Dean trap with condenser were placed 119.4 g of methyl stearate, 41 g of 3-amino-2,2-dimethylpropanol and 3 g of potassium *tert*-butoxide. The mixture was heated to 190°C, and the methanol distilled off. The reaction mixture was dissolved in hot isopropanol and filtered. On cooling, the product crystallized, and after filtration and drying it melted at 44°C. IR showed an ester carbonyl. The pure product was obtained by washing with hot hexane. The pure product melted at 68—69°C.

Example 36

*2,2-Dimethyl-3-stearamidopropyl 3-(3,5-di-tert-butyl-4-hydroxyphenyl)propionate*

In a 1-liter three-neck, round-bottom flask equipped with a thermometer stirrer, dropping funnel and condenser were placed 36.9 g of N-(2,2-dimethyl-3-hydroxypropyl)-stearamide, 25 ml of triethylamine and 250 ml of toluene. To this stirred mixture was added 29.6 g of 3-(3,5-di-*tert*-butyl-4-hydroxyphenyl)propionyl chloride. The mixture was filtered, stripped, and the residue was dissolved in hot methanol. The 2,2-dimethyl-3-stearamidopropyl stearate, melting at 60—62°C was isolated. The methanol solution was stripped, and the residue was dissolved in acetonitrile. The title compound was isolated; it melted at 44°C.

Example 37

*N,N'-bis[2,2-dimethyl-3-hydroxypropyl] oxamide*

In a 0.1-liter round-bottom flask were placed 12.0 g of 3-amino-2,2-dimethylpropanol and 20 ml of ethanol. To this stirred solution was added 8.5 g of diethyl oxalate, and the mixture was stirred. An exotherm was observed. On cooling, the title compound separated. It was removed by filtration and recrystallized from ethanol. The title product melted at 125—126°C.

Example 38

*3-Oxalyldiamidobis[2,2-dimethylpropyl 3-(3,5-di-tert-butyl-4-hydroxyphenyl)propionate]*

In a 1-liter three-neck, round-bottom flask equipped with a thermometer sdtirrer, dropping funnel and condenser were placed 13 g of N,N'-bis(2,2-dimethyl-3-hydroxypropyl)oxamide, 25 ml of triethylamine and 250 ml of toluene. To this stirred solution was added 29.6 g of 3-(3,5-di-*tert*-butyl-4-hydroxyphenyl)propionyl chloride. The mixture was stirred for four hours, filtered and stripped. The residue was heated with hot hexane. The title compound separated immediately. It was removed by filtration, and it melted at 143—144°C.

Example 39

*2-Stearamidoethyl 3,5-di-tert-butyl-4-hydroxybenzoate*

A mixture of 25 g (0.1 mole) 3,5-di-*tert*-butyl-4-hydroxybenzoic acid, 50 ml toluene and 8.5 ml thionyl chloride was refluxed for 3 hours. The excess thionyl chloride and the toluene were removed on a rotary evaporator. The acid chloride was dissolved in 50 ml toluene and was added to a mixture of 29.4 g (0.09 mole) N-(2-hydroxyethyl)stearamide, 20 ml toluene and 15 ml pyridine. The mixture was refluxed for two hours. The reaction mixture was then cooled, quenched with water, washed with dilute hydrochloric acid and with water. The toluene was removed by distillation, and the residue was crystallized from hexane. The title compound thus obtained melted at 56—58°C.

Example 40

This example shows the usefulness of the compounds of the present invention in polypropylene. It also shows the beneficial effect observed by using the compounds of the present invention in conjunction with a co-stabilizer. The particular compound used in each run of this Example and of the succeeding Examples is identified by the number of one of the above Examples in which the preparation of the compound is described.

The stabilizers were incorporated into Profax 6501 (trademark) polypropylene resin on a mill at 166°C. Seventy-five mil plaques (discs approximately one inch in diameter and 75 mil (1.9 mm) thick) were prepared by compression molding in a press at 27,000 psi (186 MPa) and 177°C. These specimens were placed in a forced air oven at 149°C, and the number of days required for embrittlement to occur was noted. When two out of three buttons embrittled (that is, they became granular due to heat aging), the specimen was considered to have failed. The results are shown in Table 1.

TABLE 1

| Compound/Co-Stabilizer | Concentration* | Days to Failure |
|---|---|---|
| none | — | 1 |
| DSTDP | 0.2 | 4 |
| Example 3 | 0.2 | 41 |
| Example 3/DSTDP** | 0.1/0.1 | 36 |
| Example 7 | 0.2 | 32 |
| Example 7/DSTDP | 0.1/0.1 | 25 |
| Example 8 | 0.2 | 34 |
| Example 8/DSTDP | 0.1/0.1 | 61 |
| Example 13 | 0.2 | 42 |
| Example 13/DSTDP | 0.1/0.1 | 59 |
| Example 14 | 0.2 | 39 |
| Example 14/DSTDP | 0.1/0.1 | 60 |
| Example 15 | 0.2 | 34 |
| Example 15/DSTDP | 0.1/0.1 | 55 |
| Example 17 | 0.2 | 26 |
| Example 17/DSTDP | 0.1/0.1 | 45 |
| Example 18 | 0.2 | 43 |
| Example 18/DSTDP | 0.1/0.1 | 69 |
| Example 20 | 0.2 | 48 |
| Example 20/DSTDP | 0.1/0.1 | 78 |
| Example 22 | 0.2 | 62 |
| Example 22/DSTDP | 0.1/0.1 | 82 |
| Example 23 | 0.2 | 59 |

TABLE 1 (continued)

| Compound/Co-Stabilizer | Concentration* | Days to Failure |
|---|---|---|
| Example 23/DSTDP | 0.1/0.1 | 98 |
| Example 24 | 0.2 | 28 |
| Example 24/DSTDP | 0.1/0.1 | 45 |
| Example 25 | 0.2 | 35 |
| Example 25/DSTDP | 0.1/0.1 | 48 |
| Example 26 | 0.2 | 55 |
| Example 26/DSTDP | 0.1/0.1 | 76 |
| Example 28 | 0.2 | 61 |
| Example 28/DSTDP | 0.1/0.1 | 55 |
| Example 32 | .2 | 40 |
| Example 32/DSTDP | 0.1/0.1 | 64 |
| Example 33 | 0.2 | 56 |
| Example 33/DSTDP | 0.1/0.1 | 84 |

* parts by weight per hundred parts by weight of polypropylene
**distearyl thiodipropionate

Example 41

This example shows the usefulness of the compounds of the present invention in EPDM (ethylene/propylene/5-ethylidene-2-norbornene terpolymer having an ethylene/propylene weight ratio of 67/33, an iodine number of 10 and a Mooney viscosity (ML-4 at 120°C) of 57).

The stabilizers (0.165 g were dissolved in a rubber cement containing 110 g of EPDM in 2,000 g hexane. The hexane was removed by slowly adding the cement to boiling water. The solids were removed from the boiling water by filtration and were dried on a mill for 5 minutes at 275—300°F (135—149°C).

The time necessary for a 1 g sample to absorb 20 cm of oxygen at 150°C was then determined ($T_{20}$). The results are shown in Table 2, indicating the excellent protection conveyed on the EPDM through the presence of the compounds of the present invention.

TABLE 2

| Additive | $T_{20}$ (minutes) |
|---|---|
| None | 30 |
| Example 15 | 560 |
| Example 16 | 260 |
| Example 23 | 405 |

The example shows the usefulness of the compounds of the present invention in a dynamically cured elastomeric blend of polypropylene (20 parts by weight) and ethylene-propylene dicyclopentadiene terpolymer (80 parts by weight).

The compound was milled into the blend at a concentration of 0.3 parts per hundred parts by weight of blend. Seventy-five mil (1.9 mm) plaques were compression molded, and buttons were

punched from these plaques. The buttons were placed in a forced air oven at 149°C, and the time for three of five buttons to embrittle was noted. The results are shown in Table 3.

TABLE 3

| Additive | Hours to Embrittlement |
|---|---|
| None | 80 |
| Example 23 | 275 |

Example 43

This example shows the usefulness of the compounds of the present invention in high impact polystyrene.

The antioxidant (0.25 parts by weight per hundred parts by weight of polystyrene) was milled into a high impact polystyrene stock at 138°C. Notched Izod bars were molded at 170°C. After a determination of impact strength was made, the Izod bars were ground and remolded. This procedure was repeated five times. The percent impact strength retention after five moldings is shown below in Table 4.

TABLE 4

| Antioxidant | % Impact Strength Retention |
|---|---|
| None | 58.3 |
| Example 15 | 76.2 |
| Example 16 | 66.3 |
| Example 23 | 85.2 |

Example 44

This experiment demonstrates the unexpectedly superior efficacy of the compound of Example 32 over di-2-[3-(3,5-di-*tert*-butyl-4-hydroxyphenyl)propionamide], prepared according to U.S.S.R. Patent 567,719 (Glushkova), in polypropylene. The test samples were prepared and tested essentially following the procedure of Example 35, with the results shown in Table 5:

TABLE 5

| Compound | % Level | Days to Failure |
|---|---|---|
| Example 32 | .2 | 40 |
| Example 32/DSTDP | .1/.1 | 64 |
| Prior Art | .2 | 6 |
| Prior Art/DSTDP | .1/.1 | 8 |

**Claims for the contracting states BE, CH, DE, FR, GB, NL**

1. An esteramide characterised in that it has the formula $R^1COOANHM$, wherein A is $C_2$ to $C_{12}$ alkylene, $C_4$ to $C_8$ cycloalkylene or $—CH_2—C(CH_2)_p$, wherein p is an integer from 3 to 6, or A is $C_2$ to $C_5$ alkylene substituted with one or two $—CH_2OCO(CH_2)_mR^3$ groups, wherein $R^3$ is 3-W-5-Y-4-hydroxyphenyl, wherein W and Y may be the same or different and are $C_1$ to $C_{12}$ alkyl and m is 0,1 or 2; $R^1$ is $—(CH_2)_mR^3$, wherein m and $R^3$ are as defined above, and M is either hydrogen or $—COR^2$, wherein $R^2$ is $C_1$ to $C_{20}$ alkyl, $C_6$ to $C_{10}$ aryl or phenyl substituted with halogen, $C_1$ to $C_{12}$ alkyl, $C_1$ to $C_{12}$ alkoxy, nitro or hydroxy or $R^2$ is $—BCONHAOCOR^1$ wherein A and $R^1$ are as defined above and B is a single bond, $C_1$ to $C_{10}$ alkylene, phenylene, $C_4$ to $C_{12}$ oxydialkylene or $C_4$ to $C_{12}$ thiodialkylene, or $R^2$ is the same

as $R^1$ which is defined above, with the proviso that B may be a single bond only if A is $C_2$ to $C_5$ alkylene substituted with one or two $—CH_2OCO(CH_2)_mR^3$ groups.

2. An esteramide according to claim 1, characterised in that A is $C_2$ to $C_5$ alkylene, cyclohexylene or $C_2$ to $C_4$ alkylene substituted with one or two $—CH_2OCO(CH_2)_mR^3$ groups, $R^1$ is $—(CH_2)_m—R^3$, wherein m is 2, W is tert-butyl, Y is methyl or tert-butyl, $R^2$ is $C_1$ to $C_{18}$ alkyl, phenyl or phenyl substituted with a $C_1$ to $C_4$ alkyl, or hydroxy or $R^2$ is $—BCONHAOCOR^1$, wherein A and $R^1$ are as defined above and B is a single bond subject to the proviso of claim 1, $C_1$ to $C_8$ alkylene, phenylene, $C_4$ thiodialkylene, or $R^2$ is the same as $R^1$ which is defined above.

3. An esteramide according to claim 2, characterised in that B is as defined in claim 2, with the proviso that B may be a single bond only when A is $C_2$ to $C_4$ alkylene substituted with one or two $CH_2OCO(CH_2)_mR^3$ groups, wherein m and $R^3$ are as defined in claim 2.

4. An esteramide according to claim 1, characterised in that B is as defined in claim 1, with the proviso that B may be a single bond only when A is $C_2$ to $C_5$ alkylene substituted with one or two $—CH_2O—CO(CH_2)_mR^3$ groups, where m and $R^3$ are as defined in claim 1.

5. A composition characterised in that it comprises an organic material subject to oxidative degradation and an oxidative degradation inhibiting amount of an esteramide according to any one of claims 1 to 4.

6. A method of inhibiting the oxidative degradation of an organic material characterised in that the method comprises adding to said material an oxidative degradation inhibiting amount of an esteramide according to any one of claims 1 to 4.

7. A method according to claim 6, characterised in that M is $—COR^2$.

**Claims for the Contracting State: AT**

1. Use of an esteramide to inhibit the oxidative degradation of an organic material characterised in that the esteramide has the formula $R^1COOANHM$, wherein A is $C_2$ to $C_{12}$ alkylene, $C_4$ to $C_8$ cycloalkylene or $—CH_2—(CH_2)_p—$ wherein p is an integer from 3 to 6, or $A_3$ is $C_2$ to $C_5$ alkylene substituted with one or two $—CH_2OCO(CH_2)_mR^3$ groups, wherein $R^3$ is 3-W-5-Y-4-hydroxyphenyl, wherein W and Y may be the same or different and are $C_1$ to $C_{12}$ alkyl and m is 0, 1 or 2; $R^1$ is $—(CH_2)_mR^3$, wherein m and $R^3$ are as defined above, and M is either hydrogen or $—COR^2$, wherein $R^2$ is $C_1$ to $C_{20}$ alkyl, $C_6$ to $C_{10}$ aryl or phenyl substituted with halogen, $C_1$ to $C_{12}$ alkyl, $C_1$ to $C_{12}$ alkoxy, nitro or hydroxy or $R^2$ is $—BCONHAOCOR^1$ wherein A and $R^1$ are as defined above and B is a single bond, $C_1$ to $C_{10}$ alkylene, phenylene, $C_4$ to $C_{12}$ oxydialkylene or $C_4$ to $C_{12}$ thiodialkylene, or $R^2$ is the same as $R^1$ which is defined above.

2. Use of an esteramide according to claim 1 characterised in that A is $C_2$ to $C_5$ alkylene, cyclohexylene or $C_2$ to $C_4$ alkylene substituted with one or two $—CH_2OCO(CH_2)_mR^3$ groups, $R^1$ is $—(CH_2)_m—R^3$, wherein m is 2, W is tert-butyl, Y is methyl or tert-butyl, $R^2$ is $C_1$ to $C_{18}$ alkyl, phenyl or phenyl substituted with a $C_1$ to $C_4$ alkyl, or hydroxy or $R^2$ is $—BCONHAOCOR^1$, wherein A and $R^1$ are as defined above and B is a single bond, $C_1$ to $C_8$ alkylene, phenylene, $C_4$ thiodialkylene, or $R^2$ is the same as $R^1$ which is defined above.

3. Use of an esteramide according to claim 2 characterised in that B is as defined in claim 2, with the proviso that B may be a single bond only when A is $C_2$ to $C_4$ alkylene substituted with one or two $CH_2OCO(CH_2)_mR^3$ groups, wherein m and $R^3$ are as defined in claim 2.

4. Use of an esteramide according to claim 1 characterised in that B is as defined in claim 1, with the proviso that B may be a single bond only when A is $C_2$ to $C_5$ alkylene substituted with one or two $—CH_2O—CO(CH_2)_mR^3$ groups, where m and $R^3$ are as defined in claim 1.

5. A composition characterised in that it comprises an organic material subject to oxidative degradation and an oxidative degradation inhibiting amount of an esteramide as defined in any of claims 1 to 4.

6. A method of inhibiting the oxidative degradation of an organic material characterised in that the method comprises adding to said material an oxidative degradation inhibiting amount of an esteramide as defined in any one of claims 1 to 4.

**Claims for the Contracting State: IT**

1. An esteramide characterised in that it has the formula $R^1COOANHM$, wherein A is $C_2$ to $C_{12}$ alkylene, $C_4$ to $C_8$ cycloalkylene or $—CH_2—(CH_2)_p$, wherein p is an integer from 3 to 6, or A is $C_2$ to $C_5$ alkylene substituted with one or two $—CH_2OCO(CH_2)_mR^3$ groups, wherein $R^3$ is 3-W-5-Y-4-hydroxyphenyl, wherein W and Y may be the same or different and are $C_1$ to $C_{12}$ alkyl and m is 0,1 or 2; $R^1$ is $—(CH_2)_mR^3$, wherein m and $R^3$ are as defined above, and M is either hydrogen or $—COR^2$, wherein $R^2$ is $C_1$ to $C_{20}$ alkyl, $C_6$ to $C_{10}$ aryl or phenyl substituted with halogen, $C_1$ to $C_{12}$ alkyl, $C_1$ to $C_{12}$ alkoxy, nitro or hydroxy or $R^2$ is $—BCONHAOCOR^1$ wherein A and $R^1$ are as defined above and B is a single bond, $C_1$ to $C_{10}$ alkylene, phenylene, $C_4$ to $C_{12}$ oxydialkylene or $C_4$ to $C_{12}$ thiodialkylene, or $R^2$ is the same as $R^1$ which is defined above.

2. An esteramide according to claim 1, characterised in that A is $C_2$ to $C_5$ alkylene, cyclohexylene or $C_2$ to $C_4$ alkylene substituted with one or two $—CH_2OCO(CH_2)_mR^3$ groups, $R^1$ is $—(CH_2)_m—R^3$, wherein m is 2, W is tert-butyl, Y is methyl or tert-butyl, $R^2$ is $C_1$ to $C_{18}$ alkyl, phenyl or phenyl substituted with a $C_1$ to $C_4$ alkyl, or hydroxy or $R^2$ is $—BCONHAOCOR^1$, wherein A and $R^1$ are as defined above and B is a single bond, $C_1$ to $C_8$ alkylene, phenylene, $C_4$ thiodialkylene, or $R^2$ is the same as $R^1$ which is defined above.

3. An esteramide according to claim 2, characterised in that B is as defined in claim 2, with the proviso that B may be a single bond only when A is $C_2$ to $C_4$ alkylene substituted with one or two $CH_2OCO(CH_2)_mR^3$ groups, wherein m and $R^3$ are as defined in claim 2.

4. An esteramide according to claim 1, characterised in that B is as defined in claim 1, with the proviso that B may be a single bond only when A is $C_2$ to $C_5$ alkylene substituted with one or two $—CH_2O—CO(CH_2)_mR^3$ groups, where m and $R^3$ are as defined in claim 1..

5. A composition characterised in that it comprises an organic material subject to oxidative degradation and an oxidative degradation inhibiting amount of an esteramide according to any one of claims 1 to 4.

6. A method of inhibiting the oxidative degradation of an organic material characterised in that the method comprises adding to said material an oxidative degradation inhibiting amount of an esteramide according to any one of claims 1 to 4.

7. A method according to claim 6, characterised in that M is $—COR^2$.

**Patentansprüche für die Vertragsstaaten BE, CH, DE, FR, GB, NL:**

1. Esteramid, dadurch gekennzeichnet, daß es die Formel $R^1COOANHM$ hat, worin A eine Alkylengruppe mit 2 bis 12 C-Atomen, eine Cycloalkylengruppe mit 4 bis 8 C-Atomen oder $—CH_2—(CH_2)_p—$ ist, worin p eine ganze Zahl von 3 bis 6 ist, oder worin A eine mit einer oder zwei $—CH_2OCO(CH_2)_mR^3$-Gruppe(n), worin $R^3$ eine 3-W-5-Y-4-Hydroxyphenylgruppe, worin W und Y gleich oder verschieden sein können und Alkyl mit 1 bis 12 C-Atomen bedeuten, und m 0, 1 oder 2 ist, substituierte Alkylengruppe mit 2 bis 5 C-Atomen ist, worin $R^1—(CH_2)_mR^3$ ist, worin m und $R^3$ die vorstehend definierte Bedeutung haben, und worin M entweder Wasserstoff oder $—COR^2$ ist, worin $R^2$ eine Alkylgruppe mit 1 bis 20 C-Atomen, eine Arylgruppe mit 6 bis 10 C-Atomen oder eine mit Halogen, Alkyl mit 1 bis 12 C-Atomen, Alkoxy mit 1 bis 12 C-Atomen, Nitro oder Hydroxy substituierte Phenylgruppe ist oder worin $R^2$ $—BCONHAOCOR^1$ ist, worin A und $R^1$ die vorstehend definierte Bedeutung haben und B eine Einfachbindung, eine Alkylengruppe mit 1 bis 10 C-Atomen, eine Phenylengruppe, eine Oxydialkylengruppe mit 4 bis 12 C-Atomen oder eine Thiodialkylengruppe mit 4 bis 12 C-Atomen ist, oder worin $R^2$ die gleiche Bedeutung wie der vorstehend definierte Rest $R^1$ hat, und zwar unter der Bedingung, dass B nur dann eine Einfachbindung sein kann, wenn A eine mit einer oder zwei $—CH_2O—CO(CH_2)_mR^3$-Gruppe(n) substituierte Alkengruppe mit 2 bis 5 C-Atomen ist.

2. Esteramid nach Anspruch 1, dadurch gekennzeichnet, daß A eine Alkylengruppe mit 2 bis 5 C-Atomen, eine Cyclohexylengruppe oder eine mit einer oder zwei $—CH_2OCO(CH_2)_mR^3$-Gruppe(n) substituierte Alkylengruppe mit 2 bis 4 C-Atomen ist, $R^1$ $—(CH_2)_m—R^3$ ist, worin m 2 ist, W t-Butyl ist, Y Methyl oder t-Butyl ist und $R^2$ eine Alkylgruppe mit 1 bis 18 C-Atomen, eine Phenylgruppe oder eine mit einer Alkylgruppe mit 1 bis 4 C-Atomen oder einer Hydroxylgruppe substituierte Phenylgruppe ist oder $R^2$ $—BCONHAOCOR^1$ ist, worin A und $R^1$ die vorstehend definierte Bedeutung haben und B eine Einfachbindung zwar unter der Bedingung nach Anspruch 1, eine Alkylengruppe mit 1 bis 8 C-Atomen, eine Phenylengruppe oder eine Thiodialkylengruppe mit 4 C-Atomen ist, oder $R^2$ die gleiche Bedeutung wie der vorstehend definierte Rest $R^1$ hat.

3. Esteramid nach Anspruch 2, dadurch gekennzeichnet, daß B die im Anspruch 2 definierte Bedeutung hat, und zwar unter der Bedingung, daß B nur dann eine Einfachbindung sein kann, wenn A eine mit einer oder zwei $—CH_2OCO(CH_2)_mR^3$-Gruppe(n), worin m und $R^3$ die im Anspruch 2 definierte Bedeutung haben, substituierte Alkylengruppe mit 2 bis 4 C-Atomen ist.

4. Esteramid nach Anspruch 1, dadurch gekennzeichnet, daß B die im Anspruch 1 definierte Bedeutung hat, und zwar unter der Bedingung, daß B nur dann eine Einfachbindung sein kann, wenn A eine mit einer oder zwei $—CH_2O—CO(CH_2)_mR^3$-Gruppe(n), worin m und $R^3$ die im Anspruch 1 definierte Bedeutung haben, substituierte Alkylengruppe mit 2 bis 5 C-Atomen ist.

5. Masse, dadurch gekennzeichnet, daß sie ein zum oxidativen Abbau neigendes, organisches Material und eine den oxidativen Abbau hemmende bzw. verzögernde Menge eines Esterämids nach einem der Ansprüche 1 bis 4 enthält.

6. Verfahren zur Hemmung bzw. Verzögerung des oxidativen Abbaus eines organischen Materials, dadurch gekennzeichnet, daß zu dem Material eine den oxidativen Abbau hemmende bzw. verzögernde Menge eines Esteramids nach einem der Ansprüche 1 bis 4 hinzugegeben wird.

7. Verfahren nach Anspruch 6, dadurch gekennzeichnet, daß M $—COR^2$ ist.

**Patentansprüche für den Vertragsstaat AT:**

1. Benutzung eines Esteramids zur Hemmung bzw. Verzögerung des oxidativen Abhaus eines organischen Materials, dadurch gekennzeichnet, dass es die Formel $R^1COOANHM$ hat, worin A eine Alkylengruppe mit 2 bis 12 C-Atomen, eine Cycloalkylengruppe mit 4 bis 8 C-Atomen oder $-CH_2-(CH_2)_p-$ ist, worin p eine ganze Zahl von 3 bis 6 ist, oder worin A eine mit einer oder zwei $-CH_2OCO(CH_2)_mR^3-$ Gruppe(n), worin $R^3$ eine 3-W-5-Y-4-Hydroxyphenylgruppe, worin W und Y gleich oder verschieden sein konnen und Alkyl mit 1 bis 12 C-Atomen bedeuten, und m 0, 1 oder 2 ist substituierte Alkylengruppe mit 2 bis 5 C-Atomen ist, worin $R^1$ $-(CH_2)_mR^3$ ist, worin m und $R^3$ die vorstehend definierte Bedeutung haben, und worin M entweder Wasserstoff oder $-COR^2$ ist, worin $R^2$ eine Alkylgruppe mit 1 bis 20 C-Atomen, eine Arylgruppe mit 6 bis 10 C-Atomen oder eine mit Halogen, Alkyl mit 1 bis 12 C-Atomen, Alkoxy mit 1 bis 12 C-Atomen, Nitro oder Hydroxy substituierte Phenylgruppe ist oder worin $R^2$ $-BCONHAOCOR^1$ ist, worin A und $R^1$ die vorstehend definierte Bedeutung haben und B eine Einfachbindung, eine Alkylengruppe mit 1 bis 10 C-Atomen, eine Phenylengruppe, eine Oxydialkylengruppe mit 4 bis 12 C-Atomen oder eine Thiodialkylengruppe mit 4 bis 12 C-Atomen ist, oder worin $R^2$ die gleiche Bedeutung wie der vorstehend definierte Rest $R^1$ hat.

2. Benutzung eines Esteramids nach Anspruch 1, dadurch gekennzeichnet, dass A eine Alkylengruppe mit 2 bis 5 C-Atomen, eine Cyclohexylengruppe oder eine mit einer oder zwei $-CH_2OCO(CH_2)_mR^3$-Gruppe(n) substituierte Alkylengruppe mit 2 bis 4 C-Atomen ist, $R^1-(CH_2)_mR^3$ ist worin m 2 ist, W t-Butyl ist, Y Methyl oder t-Butyl ist und $R^2$ eine Alkylgruppe mit 1 bis 18 C-Atomen, eine Phenylgruppe oder eine mit einer Alkylgruppe mit 1 bis 4 C-Atomen oder einer Hydroxylgruppe substituierte Phenylgruppe ist oder $R^2$ $-BCONHAOCOR^1$ ist, worin A und $R^1$ die vorstehend definierte Bedeutung haben und B eine Einfachbindung, eine Alkylengruppe mit 1 bis 8 C-Atomen, eine Phenylengruppe oder eine Thiodialkylengruppe mit 4 C-Atomen ist, oder $R^2$ die gleiche Bedeutung wie der vorstehend definierte Rest $R^1$ hat.

3. Benutzung eines Esteramids nach Anspruch 2, dadurch gekennzeichnet, daß B die im Anspruch 2 definierte Bedeutung hat, und zwar unter der Bedingung, daß B nur dann eine Einfachbindung sein kann, wenn A eine mit einer oder zwei $-CH_2OCO(CH_2)_mR^3$-Gruppe(n), worin m und $R^3$ die im Anspruch 2 definierte Bedeutung haben, substituierte Alkylengruppe mit 2 bis 4 C-Atomen ist.

4. Benutzung eines Esteramids nach Anspruch 1, dadurch gekennzeichnet, daß B die im Anspruch 1 definierte Bedeutung hat, und zwar unter der Bedingung, daß B nur dann eine Einfachbindung sein kann, wenn A eine mit einer oder zwei $-CH_2O-CO(CH_2)_mR^3$-Gruppe(n), worin m und $R^3$ die im Anspruch 1 definierte Bedeutung haben, substituierte Alkylengruppe mit 2 bis 5 C-Atomen ist.

5. Masse, dadurch gekennzeichnet, daß sie ein zum oxidativen Abbau neigendes, organisches Material und eine den oxidativen Abbau hemmende bzw. verzögernde Menge eines Esterämids nach einem der Ansprüche 1 bis 4 enthält.

6. Verfahren zur Hemmung bzw. Verzögerung des oxidativen Abbaus eines organischen Materials, dadurch gekennzeichnet, daß zu dem Material eine den oxidativen Abbau hemmende bzw. verzögernde Menge eines Esteramids nach einem der Ansprüche 1 bis 4 hinzugegeben wird.

**Patentansprüche für den Vertragsstaat IT:**

1. Esteramid, dadurch gekennzeichnet, daß es die Formel $R^1COOANHM$ hat, worin A eine Alkylengruppe mit 2 bis 12 C-Atomen, eine Cycloalkylengruppe mit 4 bis 8 C-Atomen oder $-CH_2-(CH_2)_p-$ ist, worin p eine ganze Zahl von 3 bis 6 ist, oder worin A eine mit einer oder zwei $-CH_2OCO(CH_2)_mR^3$-Gruppe(n), worin $R^3$ eine 3-W-5-Y-4-Hydroxyphenylgruppe, worin W und Y gleich oder verschieden sein können und Alkyl mit 1 bis 12 C-Atomen bedeuten, und m 0, 1 oder 2 ist, substituierte Alkylengruppe mit 2 bis 5 C-Atomen ist, worin $R^1-(CH_2)_mR^3$ ist, worin m und $R^3$ die vorstehend definierte Bedeutung haben, und worin M entweder Wasserstoff oder $-COR^2$ ist, worin $R^2$ eine Alkylgruppe mit 1 bis 20 C-Atomen, eine Arylgruppe mit 6 bis 10 C-Atomen oder eine mit Halogen, Alkyl mit 1 bis 12 C-Atomen, Alkoxy mit 1 bis 12 C-Atomen, Nitro oder Hydroxy substituierte Phenylgruppe ist oder worin $R^2$ $-BCONHAOCOR^1$ ist, worin A und $R^1$ die vorstehend definierte Bedeutung haben und B eine Einfachbindung, eine Alkylengruppe mit 1 bis 10 C-Atomen, eine Phenylengruppe, eine Oxydialkylengruppe mit 4 bis 12 C-Atomen oder eine Thiodialkylengruppe mit 4 bis 12 C-Atomen ist, oder worin $R^2$ die gleiche Bedeutung wie der vorstehend definierte Rest $R^1$ hat.

2. Esteramid nach Anspruch 1, dadurch gekennzeichnet, daß A eine Alkylengruppe mit 2 bis 5 C-Atomen, eine Cyclohexylengruppe oder eine mit einer oder zwei $-CH_2OCO(CH_2)_mR^3$-Gruppe(n) substituierte Alkylengruppe mit 2 bis 4 C-Atomen ist, $R^1$ $-(CH_2)_m-R^3$ ist, worin m 2 ist, W t-Butyl ist, Y Methyl oder t-Butyl ist und $R^2$ eine Alkylgruppe mit 1 bis 18 C-Atomen, eine Phenylgruppe oder eine mit einer Alkylgruppe mit 1 bis 4 C-Atomen oder einer Hydroxylgruppe substituierte Phenylgruppe ist oder $R^2$ $-BCONHAOCOR^1$ ist, worin A und $R^1$ die vorstehend definierte Bedeutung haben und B eine Einfachbindung, eine Alkylengruppe mit 1 bis 8 C-Atomen, eine Phenylengruppe oder eine Thiodialkylengruppe mit 4 C-Atomen ist, oder $R^2$ die gleiche Bedeutung wie der vorstehend definierte Rest $R^1$ hat.

3. Esteramid nach Anspruch 2, dadurch gekennzeichnet, daß B die im Anspruch 2 definierte Bedeutung hat, und zwar unter der Bedingung, daß B nur dann eine Einfachbindung sein kann, wenn A eine mit einer oder zwei $-CH_2OCO(CH_2)_mR^3$-Gruppe(n), worin m und $R^3$ die im Anspruch 2 definierte Bedeutung haben, substituierte Alkylengruppe mit 2 bis 4 C-Atomen ist.

4. Esteramid nach Anspruch 1, dadurch gekennzeichnet, daß B die im Anspruch 1 definierte Bedeutung hat, und zwar unter der Bedingung, daß B nur dann eine Einfachbindung sein kann, wenn A eine mit einer oder zwei $-CH_2O-CO(CH_2)_mR^3$-Gruppe(n), worin m und $R^3$ die im Anspruch 1 definierte Bedeutung haben, substituierte Alkylengruppe mit 2 bis 5 C-Atomen ist.

5. Masse, dadurch gekennzeichnet, daß sie ein zum oxidativen Abbau neigendes, organisches Material und eine den oxidativen Abbau hemmende bzw. verzögernde Menge eines Esterämids nach einem der Ansprüche 1 bis 4 enthält.

6. Verfahren zur Hemmung bzw. Verzögerung des oxidativen Abbaus eines organischen Materials, dadurch gekennzeichnet, daß zu dem Material eine den oxidativen Abbau hemmende bzw. verzögernde Menge eines Esteramids nach einem der Ansprüche 1 bis 4 hinzugegeben wird.

7. Verfahren nach Anspruch 6, dadurch gekennzeichnet, daß M $-COR^2$ ist.

**Revendications pour les Etats contractants BE, CH, DE, FR, GB, NL**

1. Estéramide, caractérisé en ce qu'il a pour formule $R^1COOANHM$, où A est un alcoylène de 2 à 12 atomes de carbone, un cycloalcoylène de 4 à 8 atomes de carbone ou $-CH_2-(CH_2)_p$, où p est un nombre entier de 3 à 6, ou bien A est un alcoylène de 2 à 5 atomes de carbone substitué par un ou deux groupes $-CH_2OCO(CH_2)_mR^3$, où $R^3$ est 3-W-5-Y-4-hydroxyphényle, où W et Y peuvent, être identiques ou différents et sont un alcoyle de 1 à 12 atomes de carbone et n est 0, 1 ou 2; $R^1$ est $-(CH_2)_mR^3$, où m et $R^3$ sont tels que définis ci-dessus et M est soit de l'hydrogène ou $-COR^2$, où $R^2$ est un alcoyle de 1 à 20 atomes de carbone, un aryle de 6 à 10 atomes de carbone ou un phényle substitué par un halogène, un alcoyle de 1 à 12 atomes de carbone, un alcoxy de 1 à 12 atomes de carbone, un nitro ou un hydroxy ou bien $R^2$ est $-BCONHAOCOR^1$ où A et $R^1$ sont tels que définis ci-dessus et B est une simple liaison, un alcoylène de 1 à 10 atomes de carbone, une phénylène, un oxydialcoylène de 4 à 12 atomes de carbone ou un thiodialcoylène de 4 à 12 atomes de carbone, ou bien $R^2$ est le même que $R^1$ qui est défini ci-dessus à condition que B puisse être une simple liaison uniquement quand A est un alcoylene de 2 à 5 atomes de carbone substitué par un ou deux groupes $CH_2OCO(CH_2)_mR^3$.

2. Estéramide selon la revendication 1, caractérisé en ce que A est un alcoylène de 2 à 5 atomes de carbone, un cyclohexylène ou un alcoylène de 2 à 4 atomes de carbone substitué par un ou deux groupes $-CH_2OCO(CH_2)_mR^3$, $R^1$ est $-(CH_2)_m-R^3$ où m est 2, W est un tert-butyle, Y est du méthyle ou du tert-butyle, $R^2$ est un alcoyle de 1 à 18 atomes de carbone, un phényle ou un phényle substitué par un alcoyle de 1 à 4 atomes de carbone ou un hydroxy ou $R^2$ est $-BCONHAOCOR^1$, où A et $R^1$ sont tels que définis ci-dessus et B est une simple liaison à condition selon la revendication 1, un alcoylène de 1 à 8 atomes de carbone, du phénylène, un thiodialcoylène à 4 atomes de carbone ou $R^2$ est le même que $R^1$ qui est défini ci-dessus.

3. Estéramide selon la revendication 2, caractérisé en ce que B est tel que défini à la revendication 2, à condition que B puisse être une simple liaison uniquement quand A est un alcoylène de 2 à 4 atomes de carbone substitué par un ou deux groupes $CH_2OCO(CH_2)_mR^3$, où m et $R^3$ sont tels que définis à la revendication 2.

4. Estéramide selon la revendication 1, caractérisé en ce que B est tel que défini à la revendication 1 à condition que B puisse être une simple liaison uniquement quand A est un alcoylène de 2 à 5 atomes de carbone substitué par un ou deux groupes $-CH_2O-CO(CH_2)_mR^3$, où m et $R^3$ sont tels que définis à la revendication 1.

5. Composition, caractérisée en ce qu'elle contient une matière organique sujette à une dégradation par oxydation et une quantité inhibant la dégradation par oxydation d'un estéramide selon l'une quelconque des revendications 1 à 4.

6. Procédé pour inhiber la dégradation par oxydation d'une matière organique, caractérisé en ce qu'il consiste à ajouter, à ladite matière, une quantité inhibant la dégradation par oxydation, d'un estéramide selon l'une quelconque des revendications 1 à 4.

7. Procédé selon la revendication 6, caractérisé en ce que M est $-COR^2$.

**Revendications pour l'Etat contractant AT**

1. Usage d'un estéramide pour inhiber la degradation par oxydation d'une matière organique, caractérisé en ce qu'il a pour formule $R^1COOANHM$, où A est un alcoylène de 2 à 12 atomes de carbone, un cycloalcoylène de 4 à 8 atomes de carbone ou $-CH_2-(CH_2)_p$, où p est un nombre entier de 3 à 6, ou bien A est un alcoylène de 2 à 5 atomes de carbone substitué par un ou deux groupes $-CH_2OCO(CH_2)_mR^3$, où $R^3$ est 3-W-5-Y-4-hydroxyphényle, où W et Y peuvent être identiques ou différents et sont un alcoyle de 1 à 12 atomes de carbone et n est 0, 1 ou 2; $R^1$ est $-(CH_2)_mR^3$, où m et $R^3$ sont tels que définis ci-dessus et M est soit de l'hydrogène ou $-COR^2$, où $R^2$ est un alcoyle de 1 à 20 atomes de carbone, un aryle de 6 à 10 atomes de carbone ou un phényle substitué par un halogène, un

alcoyle de 1 à 12 atomes de carbone, un alcoxy de 1 à 12 atomes de carbone, un nitro ou un hydroxy ou bien $R^2$ est —BCONHAOCOR$^1$ où A et $R^1$ sont tels que définis ci-dessus et B est une simple liaison, un alcoylène de 1 à 10 atomes de carbone, un phénylène, un oxydialcoylène de 4 à 12 atomes de carbone ou un thiodialcoylène de 4 à 12 atomes de carbone, ou bien $R^2$ est le même que $R^1$ qui est défini ci-dessus.

2. Usage d'un estéramide selon la revendication 1, caractérisé en ce que A est un alcoylène de 2 à 5 atomes de carbone, un cyclohexylène ou un alcoylène de 2 à 4 atomes de carbone substitué par un ou deux groupes $—CH_2OCO(CH_2)_mR^3$, $R^1$ est $—(CH_2)_mR^3$ où m est 2, W est un tert-butyle, Y est du méthyle ou du tert-butyle, $R^2$ est un alcoyle de 1 à 18 atomes de carbone, un phényle ou un phényle substitué par un alcoyle de 1 à 4 atomes de carbone ou un hydroxy ou $R^2$ est —BCONHAOCOR$^1$, où A et $R^1$ sont tels que définis ci-dessus et B est une simple liaison, un alcoylène de 1 à 8 atomes de carbone, du phénylène, un thiodialcoylène à 4 atomes de carbone ou $R^2$ est le même que $R^1$ qui est défini ci-dessus.

3. Usage d'un estéramide selon la revendication 2, caractérisé en ce que B est tel que défini à la revendication 2, à condition que B puisse être une simple liaison uniquement quand A est un alcoylène de 2 à 4 atomes de carbone substitué par un ou deux groupes $CH_2OCO(CH_2)_mR^3$, où m et $R^3$ sont tels que définis à la revendication 2.

4. Usage d'un estéramide selon la revendication 1, caractérisé en ce que B est tel que défini à la revendication 1 à condition que B puisse être une simple liaison uniquement quand A est un alcoylène de 2 à 5 atomes de carbone substitué par un ou deux groupes $—CH_2O—CO(CH_2)_mR^3$, où m et $R^3$ sont tels que définis à la revendication 1.

5. Composition, caractérisée en ce qu'elle contient une matière organique sujette à une dégradation par oxydation et une quantité inhibant la dégradation par oxydation d'un estéramide selon l'une quelconque des revendications 1 à 4.

6. Procédé pour inhiber la dégradation par oxydation d'une matière organique, caractérisé en ce qu'il consiste à ajouter, à ladite matière, une quantité inhibant la dégradation par oxydation, d'un estéramide selon l'une quelconque des revendications 1 à 4.

**Revendications pour l'Etat contractant IT**

1. Estéramide, caractérisé en ce qu'il a pour formule $R^1COOANHM$, où A est un alcoylène de 2 à 12 atomes de carbone, un cycloalcoylène de 4 à 8 atomes de carbone ou $—CH_2—(CH_2)_p$, où p est un nombre entier de 3 à 6, ou bien A est un alcoylène de 2 à 5 atomes de carbone substitué par un ou deux groupes $—CH_2OCO(CH_2)_mR^3$, où $R^3$ est 3-W-5-Y-4-hydroxyphényle, où W et Y peuvent être identiques ou différents et sont un alcoyle de 1 à 12 atomes de carbone et n est 0, 1 ou 2; $R^1$ est $—(CH_2)_mR^3$, où m et $R^3$ sont tels que définis ci-dessus et M est soit de l'hydrogène ou $—COR^2$, où $R^2$ est un alcoyle de 1 à 20 atomes de carbone, un aryle de 6 à 10 atomes de carbone ou un phényle substitué par un halogène, un alcoyle de 1 à 12 atomes de carbone, un alcoxy de 1 à 12 atomes de carbone, un nitro ou un hydroxy ou bien $R^2$ est —BCONHAOCOR$^1$ où A et $R^1$ sont tels que définis ci-dessus et B est une simple liaison, un alcoylène de 1 à 10 atomes de carbone, un phénylène, un oxydialcoylène de 4 à 12 atomes de carbone ou un thiodialcoylène de 4 à 12 atomes de carbone, ou bien $R^2$ est le même que $R^1$ qui est défini ci-dessus.

2. Estéramide selon la revendication 1, caractérisé en ce que A est un alcoylène de 2 à 5 atomes de carbone, un cyclohexylène ou un alcoylène de 2 à 4 atomes de carbone substitué par un ou deux groupes $—CH_2OCO(CH_2)_mR^3$, $R^1$ est $—(CH_2)_m—R^3$ où m est 2, W est un tert-butyle, Y est du méthyle ou du tert-butyle, $R^2$ est un alcoyle de 1 à 18 atomes de carbone, un phényle ou un phényle substitué par un alcoyle de 1 à 4 atomes de carbone ou un hydroxy ou $R^2$ est —BCONHAOCOR$^1$, où A et $R^1$ sont tels que définis ci-dessus et B est une simple liaison, un alcoylène de 1 à 8 atomes de carbone, du phénylène, un thiodialcoylène à 4 atomes de carbone ou $R^2$ est le même que $R^1$ qui est défini ci-dessus.

3. Estéramide selon la revendication 2, caractérisé en ce que B est tel que défini à la revendication 2, à condition que B puisse être une simple liaison uniquement quand A est un alcoylène de 2 à 4 atomes de carbone substitué par un ou deux groupes $CH_2OCO(CH_2)_mR^3$, où m et $R^3$ sont tels que définis à la revendication 2.

4. Estéramide selon la revendication 1, caractérisé en ce que B est tel que défini à la revendication 1 à condition que B puisse être une simple liaison uniquement quand A est un alcoylène de 2 à 5 atomes de carbone substitué par un ou deux groupes $—CH_2O—CO(CH_2)_mR^3$, où m et $R^3$ sont tels que définis à la revendication 1.

5. Composition, caractérisée en ce qu'elle contient une matière organique sujette à une dégradation par oxydation et une quantité inhibant la dégradation par oxydation d'un estéramide selon l'une quelconque des revendications 1 à 4.

6. Procédé pour inhiber la dégradation par oxydation d'une matière organique, caractérisé en ce qu'il consiste à ajouter, à ladite matière, une quantité inhibant la dégradation par oxydation, d'un estéramide selon l'une quelconque des revendications 1 à 4.

7. Procédé selon la revendication 6, caractérisé en ce que M est $—COR^2$.